(19) Europäisches Patentamt
European Patent Office
Office européen des brevets

(11) Publication number: **0 094 167**
**A2**

# (12) EUROPEAN PATENT APPLICATION

(21) Application number: **83302331.0**

(22) Date of filing: **25.04.83**

(51) Int. Cl.³: **C 07 D 405/06**, A 01 N 43/64, A 01 N 43/50 // C07D249/08, C07D233/60, C07D233/58

(30) Priority: **12.05.82 GB 8213686**

(43) Date of publication of application: **16.11.83 Bulletin 83/46**

(84) Designated Contracting States: **AT BE CH DE FR IT LI LU NL SE**

(71) Applicant: **FBC LIMITED, Hauxton, Cambridge CB2 5HU (GB)**

(72) Inventor: **Nicholass, John Franklin, 1 Hellons Park Grove, Haverhill Suffolk (GB)**

(74) Representative: **Waldman, Ralph David et al, Industrial Property Department FBC Limited Chesterford Park Research Station, Saffron Walden, Essex CB10 1XL (GB)**

(54) **Azolyl fungicide and plant growth regulators and compositions containing them.**

(57) Compounds of formula I

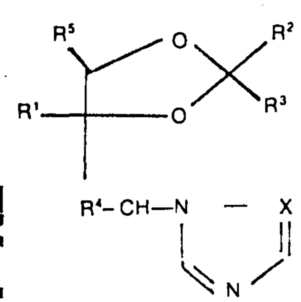

I

where $R^1$ is optionally substituted phenyl;

$R^2$ and $R^3$, which may be the same or different, are hydrogen, or alkyl of phenyl or together are $C_{4-8}$ alkylene;

$R^4$ and $R^5$, which may be the same or different, are hydrogen or alkyl and X is CH or N; together with acid addition salts, quaternary ammonium salts ad complexes of these compounds with metal salts, and certain intermediates to these, have fungicidal and plant growth regulant activity.

EP 0 094 167 A2

0094167

CASE NO BA 82/13686

AZOLYL FUNGICIDE AND PLANT GROWTH REGULATORS AND COMPOSITIONS CONTAINING THEM.

This invention relates to compounds having use in agriculture.

In G.B. patent No 1,522,657, there are described compounds having fungicidal and plant growth regulant activity in which the methyl group of 1-methyl-1H-1,2,4-triazole is substituted by a 1,3-dioxolanyl-2-yl group having a 2-aryl substituent. We have now devised methods which are different from those described in this British patent which lead to 1-methyl-1H-1,2,4-triazoles and 1-methyl-1H-imidazoles in which the methyl group is substituted by a 1,3-dioxolanyl-4-yl group. Such compounds have high fungicidal and, also in many cases, plant growth regulant activity. It could not be predicated from G.B. 1,522,657 that the compounds of the present invention would have such activity.

The invention thus provides compounds of formula I

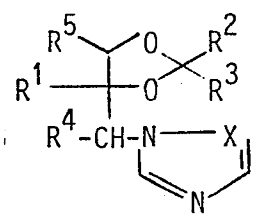

I

where $R^1$ is optionally substituted phenyl;

$R^2$ and $R^3$, which may be the same or different, are hydrogen, or alkyl or phenyl or together are $C_{4-8}$ alkylene;

$R^4$ and $R^5$, which may be the same or different, are

hydrogen or alkyl and X is CH or N; together with acid addition salts, quaternary ammonium salts and complexes of these compounds with metal salts.

The complexes are usually with a salt of formula $MA_2$ in which M is a divalent metal cation, e.g. copper, calcium or preferably manganese, and A is an anion, e.g. chloride, nitrate or a hydrocarbon sulphonate e.g. dodecylbenzenesulphonate. The molar ratio of the compound to metal salt is usually 2 or 4 to 1. Acid addition salts are usually formed with strong inorganic or organic acids e.g. hydrochloric acid, nitric acid or oxalic acid.

When the phenyl is substituted, the substituents may include one or more halogen, optionally substituted alkyl, alkoxy, alkylthio, alkoxycarbonyl, cyano, nitro, aryl or aryloxy. Preferred substituents are halogen, especially chlorine. Any alkyl and alkoxy groups are generally of 1 to 8, e.g. 1 to 6 carbon atoms. Substituents, when present, may include halogen, especially fluorine or chlorine, alkoxy or aryl. X is preferably nitrogen. $R^5$ is preferably hydrogen.

The compounds of the invention have activity as fungicides, especially against fungal diseases of plants, e.g. mildews, such as powdery mildews and particularly barley powdery mildew (Erysiphe graminis), vine downy mildew (Plasmopara viticola), and rusts such as wheat

brown rust (*Puccinia recondita*).

The invention thus also provides a method of combating fungi at a locus infested or liable to be infested therewith, which comprises applying to the locus a compound of formula I.

Some of the compounds of the invention are effective in controlling the growth of a wide variety of crops, especially to cause height reduction without any detrimental effect on the health and vigour of the plants. The invention thus also includes a method of controlling plant growth by applying to the plant a growth regulating amount of a compound of formula I. This aspect of the invention is applicable to both mono- and dicotyledonous plants, e.g. mung beans, soybeans, barley, wheat, rice, sugarbeet, cotton, sunflowers, pot plants, such as chrysanthemums, turf grass, top fruit (e.g. apples), vegetables and woody ornamentals. In some cases, the products can encourage the formation of extra shoots or tillers e.g. on cereals.

The invention also provides an agricultural composition comprising a compound of formula I in admixture with an agronomically acceptable diluent or carrier.

The composition of the invention may of course include more than one compound of the invention

In addition the composition can comprise one or more additional active ingredients, for example compounds known to possess plant-growth regulant, herbicidal, fungicidal, insecticidal or acaricidal properties. Alternatively the compounds of the invention can be used in sequence with the other active ingredient. Fungicides which can be used in conjunction with the compounds of the present invention include maneb, zineb, mancozeb, thiram, ditalimfos, tridemorph, fenpropimorph, imazalil, propiconazole, triadimefon, triadimenol, diclobutrazol, fluotrimazole, ethirimol, fenarimol, nuarimol, triforine, pyracarbolid, tolclofos-methyl, oxycarboxin, carbendazim, benomyl, thiophanate, thiophanate-methyl, thiabendazole, propineb,metalaxyl, dicloran, dithianon, fuberidazole, dodine, chlorothalonil, cyprofuram, dichlofluanid, sulphur, copper compounds, iprodione, ziram, nabam, prochloraz (and metal complexes of these e.g. the manganese chloride complex), zineb-ethylene thiuramsulphide adduct, captan, captafol, benodanil, mepronil, carboxin, guazatine, validamycin, vinclozolin, tricyclazole, quintozene, pyrazophos, furmecyclox, propamocarb, procymidone, kasugamycin, furalaxyl, folpet, fenfuram, ofurace, etridiazole, fosethyl aluminium, methfuroxam, fenapanil, benalaxyl, dinocap, fentin acetate, fentin hydroxide, IBP, cycloheximide, binapacryl,

dodemorph, dimethirimol, bupirimate, nitrothal-isopropyl quinomethionate, bitertanol, flutoluanil, etaconazole, fenpropidine, flubenzimine and cymoxanil. Plant-growth regulants with which the products can be mixed include chlormequat, mepiquat, ethephon, paclobutrazol, dikegulac-sodium, gibberellic acid, ancymidol, maleic hydrazide, mefluidide and daminozide.

The diluent or carrier in the composition of the invention can be a solid or a liquid optionally in association with a surface-active agent, for example a dispersing agent, emulsifying agent or wetting agent. Suitable surface-active agents include anionic compounds such as a carboxylate, for example a metal carboxylate of a long chain fatty acid; an N-acylsarcosinate; mono- or di-esters of phosphoric acid with fatty alcohol ethoxylates or salts of such esters; fatty alcohol sulphates such as sodium dodecyl sulphate, sodium octadecyl sulphate or sodium cetyl sulphate; ethoxylated fatty alcohol sulphates; ethoxylated alkylphenol sulphates; lignin sulphonates; petroleum sulphonates; alkyl-aryl sulphonates such as alkyl-benzene sulphonates or lower alkylnaphthalene sulphonates, e.g. butyl-naphthalene sulphonate; salts of sulphonated naphthalene-formaldehyde condensates; salts of sulphonated phenol-formaldehyde condensates; or more complex

sulphonates such as the amide sulphonates, e.g. the sulphonated condensation product of oleic acid and N-methyl taurine or the dialkyl sulphosuccinates, e.g. the sodium sulphonate of dioctyl succinate. Nonionic agents include condensation products of fatty acid esters, fatty alcohols, fatty acid amides or fatty-alkyl- or alkenyl-substituted phenols with ethylene oxide, fatty esters of polyhydric alcohol ethers, e.g. sorbitan fatty acid esters, condensation products of such esters with ethylene oxide, e.g. polyoxyethylene sorbitan fatty acid esters, block copolymers of ethylene oxide and propylene oxide, acetylenic glycols such as 2,4,7,9-tetramethyl-5-decyn-4,7-diol, or ethoxylated acetylenic glycols. Examples of a cationic surface-active agent include, for instance, an aliphatic mono-, di-, or polyamine as an acetate, naphthenate or oleate; an oxygen-containing amine such as an amine oxide or polyoxyethylene alkylamine; an amide-linked amine prepared by the condensation of a carboxylic acid with a di- or polyamine; or a quaternary ammonium salt.

The compositions of the invention can take any form known in the art for the formulation of fungicidal, plant growth regulant and similar compounds, for example, a solution, a dispersion, an aqueous emulsion, a dusting powder, a seed dressing, a fumigant, a smoke, a

dispersible powder, an emulsifiable concentrate or granules. Moreover it can be in a suitable form for direct application or as a concentrate or primary composition which requires dilution with a suitable quantity of water or other diluent before application.

As a dispersion, the composition comprises a compound of the invention dispersed in a liquid medium, preferably water. It is often convenient to supply the consumer with a primary composition which can be diluted with water to form a dispersion having the desired concentration. The primary composition can be provided in any one of the following forms. It can be a dispersible solution which comprises a compound of the invention dissolved in a water-miscible solvent with the addition of a dispersing agent. A further alternative comprises a compound of the invention in the form of a finely ground powder in association with a dispersing agent and intimately mixed with water to give a paste or cream which can if desired be added to an emulsion of oil in water to give a dispersion of active ingredient in an aqueous oil emulsion.

An emulisifiable concentrate comprises a compound of the invention dissolved in a water-immiscible solvent together with an emulsifying agent and which is formed into an emulsion on mixing with water.

A dusting powder comprises a compound of the invention

intimately mixed with a solid pulverulent diluent, for example, kaolin.

A granular solid comprises a compound of the invention associated with similar diluents to those which may be employed in dusting powders, but the mixture is granulated by known methods. Alternatively it comprises the active ingredient adsorbed or absorbed on a pre-granular diluent, for example, Fuller's earth, attapulgite or limestone grit.

A wettable powder usually comprises the active ingredient in admixture with a suitable surfactant and an inert powder diluent such as china clay.

Another suitable concentrate, particularly when the product is a solid, is a flowable suspension concentrate which is formed by grinding the compound with water, a wetting agent and a suspending agent.

The concentration of the active ingredient in the composition of the present invention, as applied to plants is preferably within the range of 0.001 to 3.0 per cent by weight, especially 0.001 to 1.0 per cent by weight. In a primary composition the amount of active ingredient can vary widely and can be, for example, from 5 to 95 per cent by weight of the composition.

In the method of the invention the compound is generally applied to seeds, plants or their habitat. Thus, the compound can be applied directly to the soil

before, at or after drilling so that the presence of active compound in the soil can control the growth of fungi which may attack seeds. When the soil is treated directly the active compound can be applied in any manner which allows it to be intimately mixed with the soil such as by spraying, by broadcasting a solid in the form of granules, or by applying the active ingredient at the same time as drilling by inserting it in the same drill as the seeds. A suitable application rate is within the range of from 0.05 to 20 kg per hectare, more preferably from 0.1 to 10 kg per hectare.

Alternatively the active compound can be applied directly to the plant by, for example, spraying or dusting either at the time when the fungus has begun to appear on the plant or before the appearance of fungus as a protective measure. In both such cases the preferred mode of application is by foliar spraying. It is generally important to obtain good control of fungi in the early stages of plant growth as this is the time when the plant can be most severely damaged. For cereal crops such as wheat, barley and oats it is often desirable to spray the plant at or before growth stage 5 although additional treatments by spraying when the plant is more mature can augment resistance to the growth or spread of fungi. The spray or dust can conveniently contain a pre- or

0094167

post-emergence herbicide if this is thought necessary. Sometimes, it is practicable to treat the roots of a plant before or during planting, for example, by dipping the roots in a suitable liquid or solid composition. When the active compound is applied directly to the plant a suitable rate of application is from 0.01 to 10 kg. per hectare, preferably from 0.05 to 5 kg. per hectare.

When the compounds of the invention are used as growth regulators they are usually best applied during the vegetative stages of growth at similar rates as above. In the case of crops such as legumes, cotton and sunflowers, application preferably takes place during the late vegetative stage of growth, just prior to or just after the onset of flowering. In the case of cereals, pot plants and turf, earlier application, during the early vegetative stage of growth, is more appropriate.

The compounds of the invention may be prepared by reacting a compound of formula II

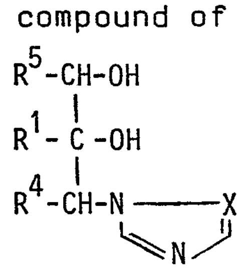

II

with a compound of formula $R^2R^3CO$. This reaction is usually carried out at a temperature of 50 to 150$^oC$, and generally under reflux. Complexes with metal salts can be

prepared by mixing the compound of formula I with the metal salt, usually in solution.

The compounds of formula II may be prepared by dihydroxylating a compound of formula III

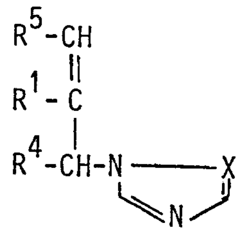

III

A suitable dihydroxylating agent is osmium tetroxide. This reaction is usually carried out with a catalytic amount of this reagent in the presence of a tertiary amine-N-oxide, in the absence of oxygen and at a temperature of 50-150°C, generally under reflux.

An alternative dihydroxylating agent is potassium permanganate, which is generally used in the presence of a phase transfer agent.

The compounds of formula III as well as their complexes with metal salts have fungicidal activity. The invention thus also provides a method of combating fungi at a locus infested or liable to be infested therewith which comprises applying to the locus a compound of formula III or a metal salt complex thereof.

Many of the compounds of formula III are novel and the invention also includes such compounds and their complexes with metal salts with the proviso that when the compound

is not a complex, $R^1$ is not phenyl or halophenyl when $R^4$ and $R^5$ are hydrogen and X is nitrogen. The invention also includes fungicidal compositions comprising these novel compounds.

Compounds of formula III may be prepared by reacting 1,2,4-triazole or imidazole with a compound of formula IV

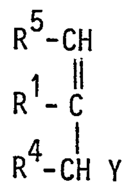

IV

in which Y is a leaving group, e.g. halogen, preferably bromine, or an aryl or alkylsulphonate. This reaction is generally carried out under basic conditions e.g. in the presence of an alkali metal carbonate or alkoxide at a temperature of e.g. -10 to 70$^{o}$C.

Complexes are usually formed in a similar manner to the formation of the complexes of the compound of formula I. The compounds of formula III and their complexes may be used and formulated in a similar manner to the compounds of formula I.

The invention is illustrated in the following Examples. Structures of isolated novel compounds were confirmed by elemental and/or other appropriate analysis.

Example 1

A mixture of 1-chloro-4-(1-methylethenyl) benzene (107g) and carbon tetrachloride (40 ml) was added to a stirred mixture of N-bromosuccinimide (124-5 g) and 2,2'-azobis (2-methylpropionitrile) (0.6 g) and the mixture heated to reflux. The stirring was stopped to allow an exothermic reaction to develop, whereupon heating was stopped and stirring continued until the reaction stopped. The mixture was allowed to cool to 70°C and hexane (700 ml) was added with stirring. The mixture was then maintained at 10°C for 30 minutes, filtered, the precipitate washed with hexane, and the filtrate and the washings evaporated to give crude 1-[1-(bromomethyl)ethenyl]-4-chlorobenzene.

This crude product (175.3 g) was added dropwise to a

stirred and cooled mixture of 1,2,4-triazole (22-8 g), potassium carbonate (50 g) and dimethylformamide (250 ml). The mixture was then stirred at room temperature for 2 hours, poured into water and extracted with ether. The extract was washed with water, evaporated to low volume and hydrochloric acid (20%, 100 ml) added. The mixture was filtered and the precipitate washed with ether. The ether layer from the filtrate was extracted with hydrochloric acid (20%, 100 ml) and the resulting combined aqueous layers washed with ether, and combined with the precipitate. This mixture was made alkaline with aqueous sodium hydroxide (40%) and extracted with ether. The extract was washed with water, dried and evaporated. The residue was recrystallised from diisopropyl ether to give 1-(2-(4-chlorophenyl)-prop-2-enyl)-1H-1,2,4-triazole, m.p. 56-8°C.

A mixture of this product (30.1 g), trimethylamine N-oxide dihydrate (20.7 g), pyridine (11 ml), t-butanol (260 ml) and deionised water (85 ml) was stirred under nitrogen and osmium tetroxide (4.4 ml of a 2.5% w/v solution in t-butanol) added. The mixture was heated under reflux for 90 hours, cooled to room temperature and aqueous sodium metabisulphate (120 ml of 20% solution) added. The mixture was evaporated to low volume, saturated aqueous sodium chloride added (100 ml) and

extracted with ethyl acetate. The extract was washed with water, dried, evaporated under reduced pressure and the residue extracted with boiling toluene to give 1-(2-(4-chlorophenyl)-2,3-dihydroxypropyl)-1*H*-1,2,4-triazole, m.p. 115-8°C.

A mixture of this product (7.4 g), isobutyraldehyde (6.5 g), *p*-toluenesulphonic acid monohydrate (0.7 g) and toluene (50 ml) was heated under reflux in a Dean and Stark apparatus for 3 hours. The mixture was then cooled, diluted with ether and washed with aqueous sodium hydroxide (5 %) and water, dried and evaporated. The residue was distilled to give 1-[[4-(4-chlorophenyl)-2-isopropyl-1,3-dioxolan-4-yl]methyl]-1*H*-1,2,4-triazole, b.p. 144 - 51°C/0.16 mm. (Compound 1).

In a similar manner the following were obtained. In some cases the propenyl triazole was obtained from the corresponding 1-(chloromethyl) ethenyl compound which itself was prepared by chlorinating the 1-(hydroxymethyl) ethenyl compound, using carbon tetrachloride and triphenyl phosphine. In some cases potassium permanganate in association with triethylbenzylammonium chloride was used as the dihydroxylating agent.

Intermediates were not always isolated and characterised. In some cases free bases were converted to a salt by treatment with a suitable acid in an organic

solvent. In the majority of such cases only the salt was characterised and tested.

Compounds were usually obtained as a mixture of cis and trans isomers. They can be separated by silica-gel chromatography. Occasionally a single isomer is obtained by recrystallisation.

In the notes column the letters have the following meanings:

A = nitrate salt

B = oxalate salt

C = hydrochloride salt

D = single isomer

E = cis-isomer

F = trans-isomer

H = prepared by treatment of 4-Br compound using cuprous cyanide.

J = Bulb to bulb distilled

K = 5-Me substituent on dioxolanyl ring

Compounds obtained are of the general formula

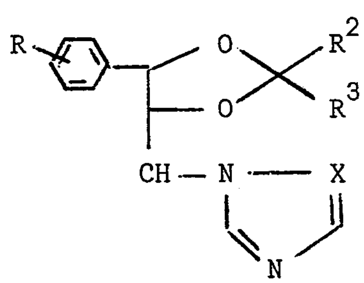

0094167

| Cpd No | X | R | $R^2$ | $R^3$ | Physical Constant m.p.(°C) | b.p. (°C/mm Hg) | Notes |
|---|---|---|---|---|---|---|---|
| 2 | N | 4-Cl | Et | Me | | 148-153/0.15 | |
| 3 | N | 4-Cl | Bu | H | | 176-78/0.3 | |
| 4 | N | H | $Bu^t$ | Me | | 132-136/0.1 | |
| 5 | N | H | Ph | H | gum | | |
| 6 | N | H | Me | Me | 69-71 | 124-126/0.2 | |
| 7 | N | 4-Me | Me | Me | 62-66 | | |
| 8 | N | 4-Me | Pr | H | | 141/0.05 | |
| 9 | N | 4-Me | $Pr^i$ | H | | 136-137/0.4 | |
| 10 | N | 4-Cl | $Pr^n$ | H | | 158-60/0.35 | |
| 11 | N | 3,4-$Cl_2$ | $Pr^n$ | H | | 165-9/0.18 | |
| 12 | N | 3,4-$Cl_2$ | $Pr^i$ | H | | 162-8/0.15 | |
| 13 | N | 4-Cl | iso-pentyl | Me | | 154-60/0.07 | |
| 14 | N | 4-Cl | Et | H | | 150/0.1 | |

| Cpd No | X | R | $R^2$ | $R^3$ | Physical Constant m.p.(°C) | b.p. (°C/mm Hg) | Notes |
|---|---|---|---|---|---|---|---|
| 15 | N | 4-Cl | Me | H | | 139-42/0.11 | |
| 16 | N | 4-Cl | $Pr^n$ | $Pr^n$ | | 154-6/0.08 | |
| 17 | N | 2,4-$Cl_2$ | $Pr^n$ | H | | 158-62/0.02 | |
| 18 | N | 4-Cl | $Pr^i$ | H | 35-9 | | F |
| 19 | N | 4-Cl | $Pr^i$ | H | oil | | G |
| 20 | N | 4-Cl | $Bu^n$ | Me | | 160-1/0.45 | |
| 21 | N | 4-Cl | $-(CH_2)_5-$ | | | 172/0.2 | |
| 22 | N | 2,4-$Cl_2$ | $Pr^i$ | H | 62-72 | 154-6/0.25 | |
| 23 | N | 4-Cl | $Bu^t$ | Me | | 146-53/0.08 | |
| 24 | N | 4-Cl | $Bu^i$ | H | | 162/0.25 | |
| 25 | N | 4-F | $Pr^i$ | H | | 142-6/0.15 | |
| 26 | N | 2,4-$Cl_2$ | $Pr^i$ | Me | | 170-6/0.5 | |
| 27 | N | H | $Pr^i$ | H | | 142-4/0.1 | |

| Cpd No | X | R | $R^2$ | $R^3$ | Physical Constant m.p.(°C) | b.p. (°C/mm Hg) | Notes |
|---|---|---|---|---|---|---|---|
| 28 | N | 4-Cl | Me | Me | | 142-6/0.15 | |
| 29 | N | 2,4-$Cl_2$ | Ph | H | 115-7 | | D |
| 30 | N | H | $Bu^t$ | H | | 130-2/0.09 | |
| 31 | N | 4-Cl | $Bu^t$ | H | | 146-8/0.06 | |
| 32 | N | 4-Cl | n-pentyl | H | | 150-6/0.06 | |
| 33 | N | 4-Cl | $Pr^i$ | Me | | 157/0.15 | |
| 34 | N | 4-Cl | $Bu^i$ | Me | | 149-52/0.25 | |
| 35 | N | 4-F | $Pr^n$ | H | | 150-2/0.15 | |
| 36 | N | 2,4-$Cl_2$ | $Bu^i$ | H | | 162-4/0.1 | |
| 37 | N | H | $Bu^i$ | H | | 135-8/0.06 | |
| 38 | N | 4-Br | $Pr^n$ | H | | 158-60/0.05 | |
| 39 | N | 4-Br | cyclohexyl | H | | 186-210/0.03 | J |
| 40 | N | 4-Br | $CH_2SMe$ | Me | | 160-90/0.05 | J |

| Cpd No | X | R | $R^2$ | $R^3$ | Physical Constant m.p.(°C) | b.p. (°C/mm Hg) | Notes |
|---|---|---|---|---|---|---|---|
| 41 | N | H | $CH_2SMe$ | Me | | 160-80/0.05 | J |
| 42 | N | H | $Pr^n$ | H | | 135-8/0.06 | |
| 43 | N | 4-Br | $Pr^i$ | H | | 155-9/0.07 | |
| 44 | N | 4-Br | $Bu^i$ | H | | 182-4/0.1 | |
| 45 | N | 4-Br | Et | Me | | 168-173/0.15 | |
| 46 | N | 4-Cl | hexyl | H | | 169-70/0.04 | |
| 47 | N | $4-Bu^t$ | $Pr^i$ | H | | 142-6/0.05 | |
| 48 | N | $2,4-Cl_2$ | $Pr^i$ | Et | 90-2 | | |
| 49 | N | 4-Br | Ph | H | | oil | |
| 50 | N | $4-Bu^t$ | $Pr^n$ | H | | 157-8/0.07 | |
| 51 | N | $4-Bu^t$ | $Bu^i$ | H | | 152-6/0.07 | |
| 52 | N | $4-Bu^t$ | Et | Me | | 140-146/0.04 | |
| 53 | N | $4-Bu^t$ | Et | H | | 148-56/0.2 | |

| Cpd No | X | R | $R^2$ | $R^3$ | Physical Constant m.p.(°C) | b.p. (°C/mm Hg) | Notes |
|---|---|---|---|---|---|---|---|
| 54 | N | 2,4-$Cl_2$ | cyclohexyl | H | | 180-200/0.04 | J |
| 55 | N | 2,5-$Cl_2$ | $Pr^n$ | H | | 155-7/0.05 | |
| 56 | N | 2-Cl | $Pr^n$ | H | | 137-41/0.03-4 | |
| 57 | N | 3-Cl | Et | Me | | 132-5/0.03 | |
| 58 | N | 2-Cl | Et | Me | | 131-2/0.03 | |
| 59 | N | 3-Cl | $Pr^n$ | H | 92-4 | 139-50/0.03 | |
| 60 | N | 4-Cl | 4-Cl-Ph | H | 158-9 | | A,D |
| 61 | N | 2-Cl | Et | H | 115-7 | | A |
| 62 | N | 2-Cl | $Pr^i$ | H | 117-20 | | A |
| 63 | N | 4-Cl | Et | Et | 63-4.5 | 146-50/0.07 | |
| 64 | N | 4-Cl | $Et_2CH$ | H | | 154-8/0.07 | |

0094167

| Cpd No | X | R | $R^2$ | $R^3$ | Physical Constant m.p.(°C) | b.p. (°C/mm Hg) | Notes |
|---|---|---|---|---|---|---|---|
| 65 | N | H | Et | Et | oil | | |
| 66 | N | $2,4-Cl_2$ | Et | Et | 106.5-7.5 | | |
| 67 | N | 4-Cl | cyclopropyl | Me | | 155-60/0.1 | |
| 68 | N | $3-CF_3$ | $Pr^i$ | H | | 123-9/0.05 | |
| 69 | N | $3-CF_3$ | Et | Et | 71-4 | | |
| 70 | N | $2,4-Cl_2$ | $Pr^n$ | H | 151-3 | | A,D |
| 71 | N | $3-CF_3$ | Me | Me | 79-84 | | |
| 72 | CH | 4-Cl | $Pr^i$ | H | 206.5-8 | | B |
| 73 | N | $2,4-Cl_2$ | Et | Me | 73-7 | | |
| 74 | N | $2,4-Cl_2$ | $Bu^t$ | H | | 155-6/0.09 | |
| 75 | N | $2,4-Cl_2$ | $Bu^t$ | Me | 101-3 | 150/0.09 | J |
| 76 | N | 4-Cl | Et | $Pr^i$ | 115-7 | | A |
| 77 | N | $2,4-Cl_2$ | Me | Me | 123-5 | | |

0094167

| Cpd No | X | R | $R^2$ | $R^3$ | Physical Constant m.p.(°C) | b.p. (°C/mm Hg) | Notes |
|---|---|---|---|---|---|---|---|
| 91 | CH | 4-MeS | $Pr^i$ | H | 180-1 | | B |
| 92 | CH | 4-Pho | $Pr^i$ | H | 144.5-6 | | B |
| 93 | B | 4-PhO | $Pr^i$ | H | 131-2.5 | | A |
| 94 | CH | 4-Cl | $-(CH_2)_4-$ | | 201-3 | | B |
| 95 | N | 4-Cl | $-(CH_2)_4-$ | | 145-6.5 | | A |
| 96 | CH | 4-Cl | $Bu^t$ | H | 199.5-201 | | B |
| 97 | CH | 4-Cl | $Pr^i$ | H | 206.5-8 | | B |
| 98 | N | 4-Br | Et | Et | 77-9 | | |
| 99 | N | 4-Cl | $Pr^i$ | H | 124-6 | | A,K |

During the preparation of the compounds of the previous Examples the following intermediates were isolated and purified.

| Cpd No | X | R | $R^2$ | $R^3$ | Physical Constant m.p.(°C) | b.p. (°C/mm Hg) | Notes |
|---|---|---|---|---|---|---|---|
| 78 | N | $3-CF_3$ | $Pr^n$ | H | | 127-32/0.6 | |
| 79 | N | $2,4-Cl_2$ | $2,4-Cl_2Ph$ | H | 168-70 | | A |
| 80 | N | 4-CN | Et | Et | 104-5 | | H |
| 81 | CH | 4-Cl | Et | Et | 162-5 | | B |
| 82 | CH | 4-Ph | $Pr^i$ | H | 158-9 | | B |
| 83 | CH | 4-Ph | Me | Me | 187-8 | | B |
| 84 | CH | 4-Cl | Me | Me | 171-3 | | B |
| 85 | CH | 4-Cl | $Pr^n$ | H | 182-3.5 | | B |
| 86 | CH | 4-Cl | Et | Me | 173-4 | | B |
| 87 | N | 4-Ph | Me | Me | 144-5 | | A |
| 88 | N | 4-Ph | $Pr^n$ | H | 139-40 | | A |
| 89 | N | 4-Ph | Et | Et | 94-6 | | A |
| 90 | N | 4-Ph | $Pr^i$ | H | 122-3 | | A |

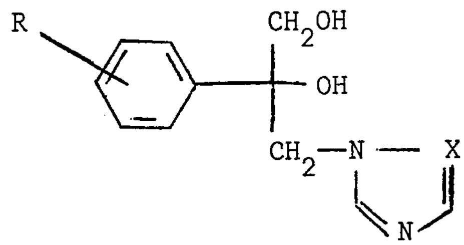

| Compound | R | X | $R^5$ | m.p. (°C) |
|---|---|---|---|---|
| a | 4-Cl | N | H | 115-8 |
| b | 2,4-$Cl_2$ | N | H | 125-6.5 |
| c | H | N | H | 108-9 |
| d | 4-Me | N | H | 140-3 |
| e | 3,4-$Cl_2$ | N | H | 116.5-118.5 |
| f | 4-F | N | H | 129-31 |
| g | 4-Br | N | H | 125.5-126.5 |
| h | 2,5-$Cl_2$ | N | H | 123-5 |
| i | 3-Cl | N | H | 108-10 |
| j | 2-Cl | N | H | 114.5-117 |
| k | 4-Cl | CH | H | 154-6 |
| l | 3-$CF_3$ | N | H | 89-90 |
| l' | 4-Cl | N | Me | 167.5-8.5 |

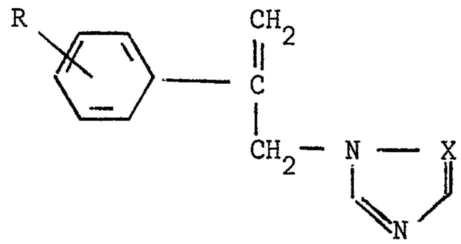

| Compound | R | X | m.p. (°C) | Notes |
|---|---|---|---|---|
| m | 4-Cl | N | 56-8 | |
| n | 4-Me | N | 56-7 | |
| o | H | N | 39-41 | |
| p | 2,4-$Cl_2$ | N | 36-37.5 | |
| q | 3,4-$Cl_2$ | N | 101-3 | |
| r | 4-Cl | CH | 61-3 | |
| s | 4-F | N | 42-4 | |
| t | 2,4-$Cl_2$ | CH | oil | |
| u | 4-Br | N | 65-7 | |
| v | 4-$Bu^t$ | N | 37-40 | |
| w | 2,5-$Cl_2$ | N | 55-6 | |
| x | 3-Cl | N | 55-7 | |
| y | 2-Cl | N | oil | |
| z | H | CH | 156-8 | C |
| aa | 3-$CF_3$ | N | 140-2 | C |

Example 2

To a solution of the product of compound 63 of Example 1 (0.2 mole) in ethanol was added an aqueous solution of cupric chloride dihydrate (0.1 mole) and the mixture stood to allow the product to crystallise. This was filtered off and washed with water, ethanol and diethyl ether to give 1-[(4-(4-chlorophenyl)-2,2-diethyl -1,3-dioxolan-4-yl)methyl]-1H-1,2,4-triazole cupric chloride complex (2:1), m.p. 158-60°C. (Complex 1)

In a similar manner complexes of the following compounds from Example 1 were obtained.

| Complex No | Compound | Ratio of organic compound to metal salt | Metal salt | m.p. (°C) |
|---|---|---|---|---|
| 2 | 65 | 2 | $CuCl_2$ | 141-3 |
| 3 | 66 | 2 | $CuCl_2$ | 171-2.5 |
| 4 | p | 2 | $CuCl_2$ | 160-3 |

| Complex No | Compound | Ratio of organic compound to metal salt | Metal salt | m.p. (°C) |
|---|---|---|---|---|
| 5 | o | 2 | $CuCl_2$ | 133-5 |
| 6 | m | 2 | $CuCl_2$ | 175-7 |
| 7 | z | 2 | $CuCl_2$ | 133.5-7 |
| 8 | z | 4 | $CuCl_2$ | 78.5-9.5 |
| 9 | r | 2 | $CuCl_2$ | 182-4 |
| 10 | 98 | 2 | $CuCl_2$ | 184-5°C |

Example 3

Aqueous acetone suspensions of the compound under test, at various concentrations, containing 125g per litre of polyoxyethylene sorbitan monolaurate and ethylene oxide-propylene oxide block copolymer wetting agents, were applied to the leaves (sprayed to "run-off") and to the soil surrounding the roots, (1ml liquid/25ml soil) of barley plants (Hordeum vulgaris) having 2 fully expanded leaves. The treated plants together with controls, treated with aqueous solutions of wetting agent only, were inoculated 24 hours later by overhead dusting with spores of barley powdery mildew (Erysiphe graminis). The plants

were placed in an atmosphere of 100% relative humidity for 24 hours and then transferred to a controlled environment room (18°C and 80-90% relative humidity) for ten days after which the disease control was assessed.

Compounds 1-47, 49-51, 55-61, 63 and 64, m-o, q, s-u and w-y and complexes 4-6, 8 and 9 gave greater than 50% control of the disease compared with the controls, at a concentration of 2000 ppm (w/v) or less.

Example 4

In similar experiments to Example 3, products were tested for activity against wheat brown rust (*Puccinia recondita*) on wheat plants (*Triticum aestivum*) having 2 fully expanded leaves - assessment being 12 days after transfer to the controlled environment room. Inoculation is with aqueous spore suspension of the pathogen.

Compounds 1-3, 4, 10-12, 14-19, 21-26, 28, 30, 31, 33, 35, 36, 39, 43, 45, 49, 51, 53, 58, s-u, and x and complexes 4 and 7-9 gave greater than 50% control of the disease compared with controls at a concentration of 500 ppm (w/v) or less.

Example 5

Mung bean seeds were sown in pots containing coarse grade vermiculite (3-5 seeds per 6cm pot). Five days later each pot was placed in approximately 100ml of an aqueous dispersion of the chemical under test and shoots

which had emerged were sprayed to run-off with a portion of test liquid. Ten days later the heights of the seedlings were measured and compared with control plants. Similar tests were also carried out on barley or wheat and on sunflower. The following compounds gave a reduction of at least 25% in height, compared with controls, at a concentration of 100mg/litre or less, without any significant adverse effects on the health and vigour of the plants.

Mung Beans

Compounds 1-10, 15, 25-28, 30, 31, 35-47, 49-53, 55-57, 59, 61-64, 77, 75, 77*, 79* and complex 1.

Barley

Compounds 14, 15, 25-47, 49-53, 55-64, 67*, 77*, 78* and complex 1.

Wheat

Compounds 1-10 and 12.

Sunflower

Compounds 2, 5-11, 14, 15, 20, 25-28, 30, 33-35, 37-45, 50-53, 55-59, 62, 64 and complex 1.

* = foliar spray only.

Example 6

This example illustrates a typical concentrate which can be formulated using compounds of the invention.

| Emulsifiable concentrate | | % w/v |
|---|---|---|
| Compound 1 | | 50 |
| Nonylphenylethoxylate | | 7 |
| Calcium dodecylbenzenesulphonate | | 3 |
| Xylene | to | 100 |

Claims

1) Compounds of formula I

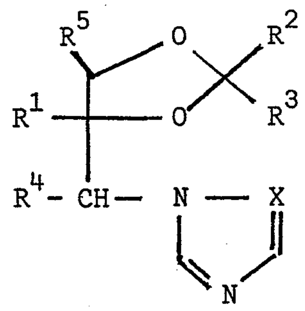

I

where $R^1$ is optionally substituted phenyl; $R^2$ and $R^3$, which may be the same or different, are hydrogen, or alkyl or phenyl or together are $C_{4-8}$ alkylene; $R^4$ and $R^5$, which may be the same or different, are hydrogen or alkyl and X is CH or N; together with acid addition salts, quaternary ammonium salts and complexes of these compounds with metal salts.

2) An agricultural composition which comprises a compound as claimed in Claim 1 in admixture with an agronomically acceptable diluent or carrier.

3) A method of combating fungi at a locus infested or liable to be infested therewith, which comprises applying to the locus a compound claimed in claim 1.

4) A method of controlling plant growth which comprises applying to the plant, a growth regulating amount of a compound claimed in claim 1.

5) A method of combating fungi at a locus infested or liable to be infested therewith, which comprises applying to the locus a compound of formula III.

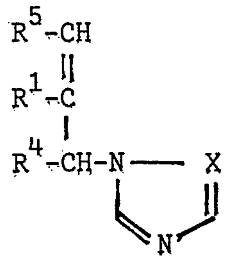

III

or a complex thereof with a metal salt, wherein $R^1$, $R^4$, $R^5$ and X have the meanings given in Claim 1.

6) Compounds of formula III as disclosed in Claim 5 and complexes thereof with a metal salt, with the proviso that when the compound is not a complex, $R^1$ is not phenyl or halophenyl when $R^4$ and $R^5$ are hydrogen and X is nitrogen.

7) An agricultural composition which comprises a compound as claimed in Claim 6 in admixture with an agronomically acceptable diluent or carrier.

OSD/RDW/MFS

I.D. 0336I